# EUROPEAN PATENT SPECIFICATION

(11) **EP 4 373 563 B1**
(45) Date of publication and mention of the grant of the patent: **04.03.2026**
(21) Application number: 22747150.5
(22) Date of filing: 08.07.2022
(51) Int. Cl.: A61N 1/04, A61N 1/36

(54) **DEVICE FOR STIMULATING A TARGET AREA**
VORRICHTUNG ZUR STIMULATION EINES ZIELBEREICHS
DISPOSITIF DE STIMULATION D'UNE ZONE CIBLE

(30) Priority: 23.07.2021 SE 2150968
(43) Date of publication of application: 29.05.2024
(73) Proprietor: Frigg AB, 18163 Lidingö (SE)
(72) Inventor: OLOFSSON, Peder, 18163 Lidingö (SE); GLOWACKI, Eric, 61200 Brno (CZ); WILLIAMSSON, Adam, 60360 Norrköping (SE)
(74) Representative: Bergenstråhle & Partners AB
(86) International application number: PCT/SE2022/050704
(87) International publication number: WO 2023/003501

(56) References cited:
- WO-A1-2017/023914
- WO-A1-2018/109715
- WO-A1-2021/044168
- LI JUNWEI ET AL: "Analytical and Experimental Investigation of Temporal Interference for Selective Neuromuscular Activation", IEEE TRANSACTIONS ON NEURAL SYSTEMS AND REHABILITATION ENGINEERING, IEEE, USA, vol. 28, no. 12, 16 November 2020 (2020-11-16), pages 3100 - 3112, XP011834468, ISSN: 1534-4320, [retrieved on 20210128], DOI: 10.1109/TNSRE.2020.3038025

## Description

### Technical Field

The present description relates generally to devices and methods for stimulating a target area. More specifically, the present description relates to devices and methods for stimulating patient tissue, especially peripheral nerves.

### Background

Nerves regulate organ function, muscle movement and balance. These functions can be therapeutically treated by electrical nerve stimulation. The human nervous system divided into the central nervous system (CNS) and the peripheral nerves (PN). Nerve stimulation can be performed on both the CNS central and peripheral nerves.

Bioelectronic therapeutics based on PN stimulation have been established for several decades and include technologies which are clinically approved in various phases of clinical trials. The most widespread PN stimulation examples are: 1. Vagus nerve stimulation (VNS), which is clinically approved for treatment of certain types of epilepsy. VNS is also in clinical trials for treatment of chronic inflammatory conditions, depression, arthritis, obesity, and many other examples. 2. Sacral nerve stimulation, which is used to treat various bowel and bladder problems. 3. Foot drop electrical stimulation, which is artificial stimulation of the nerves in the leg in order to treat foot drop, a condition where the feet "drag" and walking is difficult. This symptom is caused by a range of different nervous disorders. 4. Hypoglossal nerve to treat sleep apnea, which contracts the genioglossus muscle via stimulation of the hypoglossal nerve, causing the tongue to protrude and obstructing the airway. 5. Stimulation of the phrenic nerve, causing the diaphragm to contract and enabling breathing, permitting substitution for traditional ventilators. These PN stimulation examples are the most common and established clinically, however many other targets of PN stimulation also exist and are reported in clinical trials.

Nearly all PN stimulation technologies are based on direct injection of pulsed electrical currents into the nervous tissue to achieve stimulation. Neurostimulation involves stimulation frequencies in the range of 1-200 Hz. Peripheral nerves are located inside the body, therefore the key challenge of all PN stimulation technologies is how to effectively stimulate something at depth, below skin and tissue.

Currently, widespread solutions involve implantable stimulators. Implantable stimulators rely on established cardiac stimulator technologies, which are simply wired to the nerve directly. This represents the most common clinical solution. Naturally, such a procedure is surgically invasive, which often is a serious disadvantage. Surgery is always a risk, and batteries must be regularly replaced. This disadvantage motivates finding ways to stimulate from outside of the body in a non-invasive way.

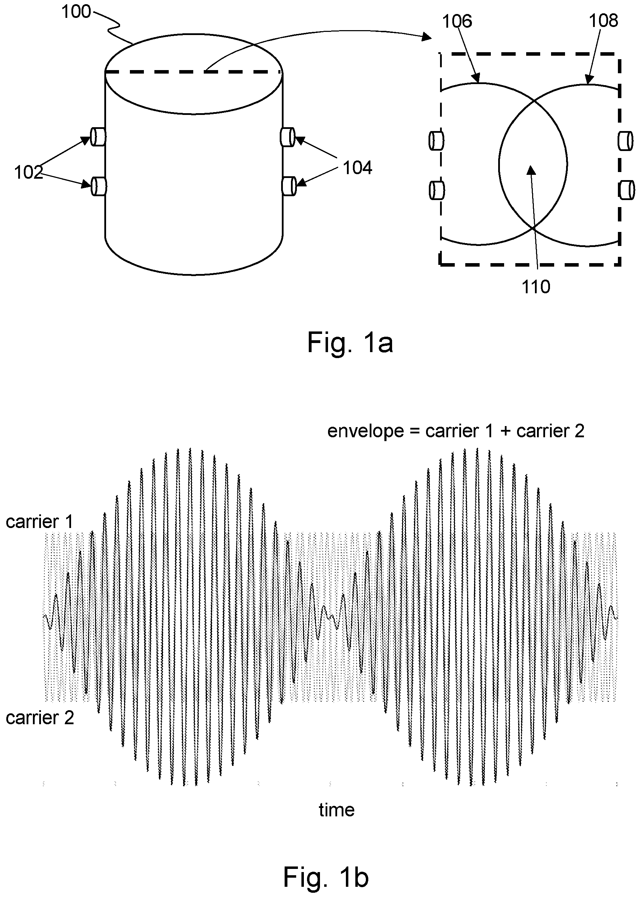

Temporal interference (TI) stimulation is a non-invasive form of electrical stimulation that has been described for use in the brain. TI relies on using relatively higher carrier frequencies (>1000 Hz). Frequencies in this range have two important properties: first, they do not elicit stimulation themselves, they pass through neural tissue without evoking effects; second, they can penetrate through all kinds of tissue, including bone, with very low loss. Essentially, the body is transparent to these electrical frequencies. Current TI stimulation have temporal interference between two carrier frequencies, while creating an envelope signal between the two carrier frequencies. For example, a 2000 Hz carrier signal and a 2010 Hz carrier signal have temporal interference, creating areas with 10 Hz envelope frequency. Therefore, this region of tissue is effectively stimulated at a frequency of 10 Hz. The TI system and method in prior art will be explained in detail in conjunction with Fig. 1.

For example, US10981003B1 discloses a system and a method employing interferential electrical stimulation following shoulder surgery. WO2019143790A1 discloses systems and methods for treating inflammatory bowel disease through peripheral nerve stimulation, wherein interferential stimulation can be utilized. WO 20170023914 A1 discloses spinal cord stimulation with interferential current.

However, current TI stimulation has two limitations: firstly, the current stimulation is used for the brain, not peripheral nerves, necessitating new parameters; secondly, the accuracy of the **TI** method in the prior art does not fulfill the requirement of accuracy of stimulating a specific peripheral nerve. There is a need for a more spatially accurate TI peripheral nerve stimulation device and method to achieve more precise peripheral nerve stimulation.

### Summary

An object of the present invention is to achieve a device, with which it is possible to perform an accurate **TI** peripheral nerve stimulation, i.e. stimulation of a target peripheral nerve. This is accomplished with an electronic device used for stimulating a target area by means of a targeting signal. The electronic device comprises at least four pairs of electrodes, i.e. at least a first, a second, a third and a fourth pair of electrodes and at least four signal generators, i.e. at least a first, a second, a third and a fourth signal generator, wherein each pair of electrodes is connected to one of the at least four signal generators and the electronic device is configured to, generate, by means of each of the at least four signal generators a total of at least four periodic carrier signals, output, by means of each pair of the at least four pairs of electrodes, the at least four periodic carrier signals, i.e. at least a first, a second, a third and a fourth periodic carrier signal; wherein, the first periodic carrier signal output by the first pair of electrodes has a first carrier frequency, the second periodic carrier signal output by the second pair of electrodes has a second carrier frequency, the third periodic carrier signal output by the third pair of electrodes has a third carrier frequency, the fourth periodic carrier signal output by the fourth pair of electrodes has a fourth carrier frequency, the first periodic carrier signal and the second periodic carrier signal cause temporal interference, and generate a first envelope signal with a first envelope frequency, wherein said first envelope frequency is equal to the frequency difference between the first carrier frequency and the second carrier frequency, the third periodic carrier signal and the fourth periodic carrier signal cause temporal interference, and generate a second envelope signal with a second envelope frequency, wherein said second envelope frequency is equal to the frequency difference between the third carrier frequency and the fourth carrier frequency, and equal to the first envelope frequency, said at least four pairs of electrodes are positioned in such a way that the first envelope signal and the second envelope signal are at least partially superimposed, and the superimposed part of the envelope signals defines the targeting signal used to stimulate the target area.

In an exemplary embodiment of the electronic device, the difference between a mean frequency used to create one envelope and a mean frequency used to create another envelope is greater than 500 Hz, preferably greater than 1kHz.

In an exemplary embodiment of the electronic device, the first envelope frequency and the second envelope frequency are within a range of 0.001-500 Hz, respectively.

In another exemplary embodiment of the electronic device, the first carrier frequency, the second carrier frequency, the third carrier frequency and the fourth carrier frequency are within a range of 500 Hz-5 GHz, respectively.

In another exemplary embodiment of the electronic device, the additional electrode pairs other than the first to fourth electrode pair, generate envelope signals for each of the additional pairs of electrodes, wherein the generated envelope signals have a common envelope frequency equal to the first envelope frequency and the second envelope frequency, the generated envelope signals are at least partially superimposed to the superimposed part of the first and second envelope signal, the common superimposed part of all the envelope signals further defines the targeting signal used to stimulate the target area.

In another exemplary embodiment of the electronic device, the target area has the shape of target cylinder.

### Brief Description of Drawings

The solution will now be described in more detail by means of exemplary embodiments and with reference to the accompanying drawings, in which:
Fig. 1a and Fig. 1b are illustrations of temporal interference in prior art.
Fig. 2 is an illustration of the electronic device used for stimulating a target area, according to exemplary embodiments.
Fig. 3 is another an illustration of the electronic device described in fig. 2 in more detail.
Fig. 4a and Fig. 4b are illustrations of envelope signals according to an exemplary embodiment.
Fig. 5 is an illustration of superimposed envelop signals, according to an exemplary embodiment.
Fig. 6 is a flow chart of a method
Fig. 7 and Fig 8 shows exemplary embodiments of arrangements of the electrodes for stimulating the sciatic nerve and the vagus nerve, with different number of electrode pairs and how the accuracy of the targeting signal increases with increasing number of electrode pairs.
Fig. 9 shows application of electrodes on the periphery of an imaginary hollow cylinder and how the accuracy is increasing with increasing number of electrode pairs.
Fig. 10 shows pair of electrodes in form of high-density grids.
Fig. 11 shows experimental data from tests performed for stimulation of the sciatic nerve and the vagus nerve, both with standard electrodes and high-density grids.

### Detailed Description

Fig. 1a shows a typical electronic device used for TI stimulation in prior art. A target object 100 is the object being stimulated by the electronic device. The electronic device comprises two pairs of electrodes 102 and 104, connected to two signal generators (not shown). For each pair of electrodes, current is injected into tissue using the two electrodes to complete the current path. The current is injected from one electrode, through the tissue, and then collected at a second electrode. The two pairs of electrodes 102 and 104 are placed adjacently on the surface of the target object 100. Each pair of electrodes is connected to one signal generator. The connection can be wired or wireless. Each signal generator generates a periodic carrier signal to the connected pair of electrodes. Each pair of electrodes output a periodic carrier signal, totally two periodic carrier signals. The electric field 106, 108 generated by the periodic carrier signals are shown in Fig. 1a. As an example, the carrier signal 106 has a frequency of 1100 Hz and the carrier signal 108 has a frequency of 1105 Hz. As Fig. 1b shows, the two periodic carrier signals have temporal interference in their overlapped area. The temporal interference generates an envelope signal with a frequency of 5 Hz. The frequency of the envelope signal is equal to the frequency difference between the two periodic carrier signals. Referring to Fig. 1a, the electric field of the envelope signal is shown as 110. The envelope signal 110 is used as a stimulation signal to stimulate the corresponding target area on the target object 100.

As described above in the background section, the high frequency carrier signal can penetrate human skin and tissue and reach in depth of the body. However, the high frequency carrier signal cannot stimulate the tissue; only lower frequency signals are effective for stimulating. Thus, an envelope signal is generated by two periodic carrier signals via temporal interference; the envelope signal has a lower frequency and can stimulate the tissue.

However, when the target area is smaller than the coverage of the envelope signal 110, the envelope signal 110 also stimulates an area outside the target area, which is an unwanted result.

This invention discloses an electronic device which can stimulate the target area more exactly. At least four pairs of electrodes are used and at least two envelope signals are generated. The envelope signals are superimposed, and the superimposed part of the envelope signals are used to stimulate the target area. This method is an improved **TI** method.

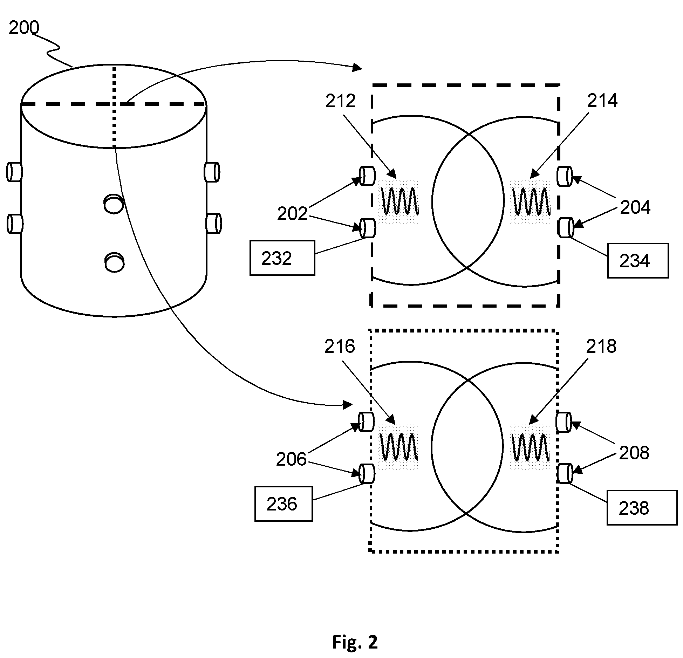

Fig. 2 shows an electronic device which can perform exact stimulation in the target area which is a part of the target object 200. The electronic device comprises at least four signal generators 232, 234, 236 and 238 and four pairs of electrodes 202, 204, 206 and 208. Every signal generator is connected to a pair of electrodes. The connection can be wired or wireless.

Each signal generator generates a periodic carrier signal and there are at least four periodic carrier signals generated. The periodic carrier signals can be a sinusoidal signal, a square-wave signal, etc. The connected pair of electrodes output the at least four generated periodic carrier signals as four periodic carrier signals 212, 214, 216 and 218. The signal generators can be e.g., current sources.

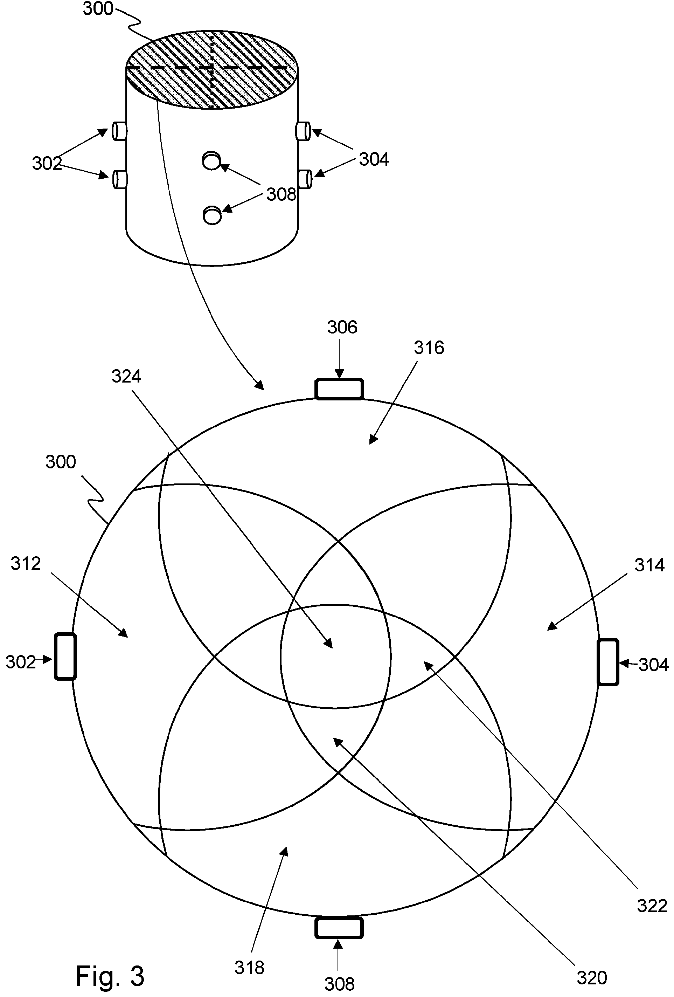

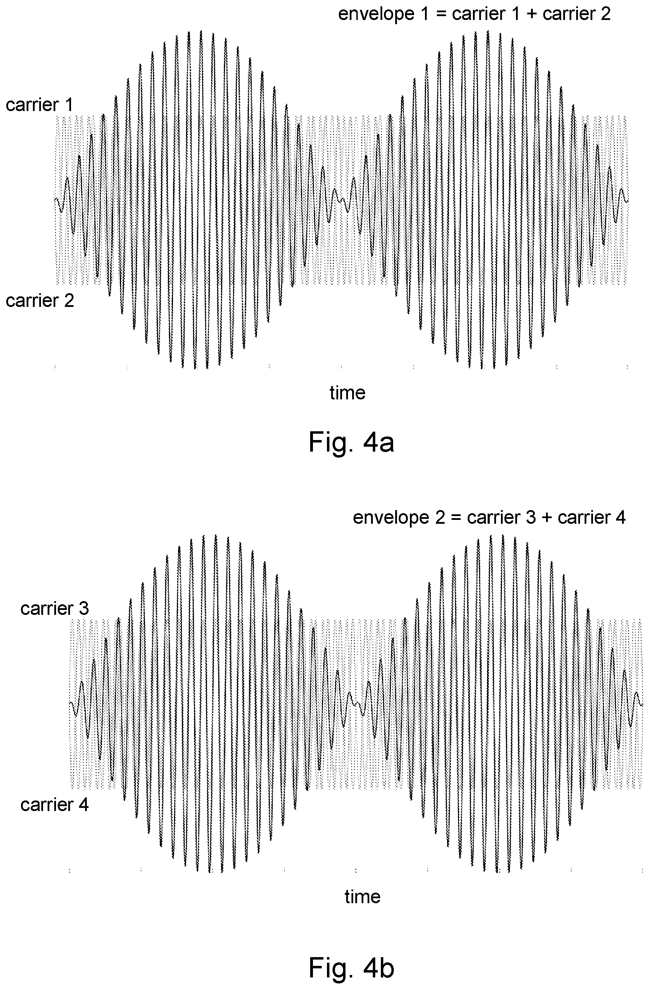

Referring to Fig. 3, which shows an embodiment with at least four signal generators (not shown in the figure), there are at least four pairs of electrodes 302, 304, 306 and 308. The electric fields of the periodic carrier signals output by the at least four electrodes are shown as 312, 314, 316 and 318. The periodic carrier signals 312 and 314 have temporal interference and generate an envelope signal. The electric field of the envelope signal has in this embodiment a rugby ball shaped area 320 in Fig. 3. Referring to Fig. 4a, the frequency of the envelope signal 320 is equal to the frequency difference between the periodic carrier signals 312 and 314. For example, the frequency of the carrier signal 312 is 1100 Hz and the frequency of the carrier signal 314 is 1105 Hz, so that the frequency of the envelope signal 320 is 5 Hz.

Similarly, the periodic carrier signals 316 and 318 have temporal interference and generate an envelope signal. The electric field of the envelope signal has a rugby ball shaped area 322 in Fig. 3. Referring to Fig. 4b, the frequency of the envelope signal 322 is equal to the frequency difference between the period carrier signals 316 and 318. For example, the frequency of the carrier signal 316 is 2100 Hz and the frequency of the carrier signal 318 is 2105 Hz, so that the frequency of the envelope signal 322 is 5 Hz. The frequency of the envelope signal 322 is equal to the frequency of the envelope signal 320.

Referring to Fig. 3, the envelope signals 320 and 322 are partially superimposed. The superimposed part is shown as 324. The superimposed part 324 of the envelope signals defines the targeting signal used to stimulate the target area. The target area can be patient tissue.

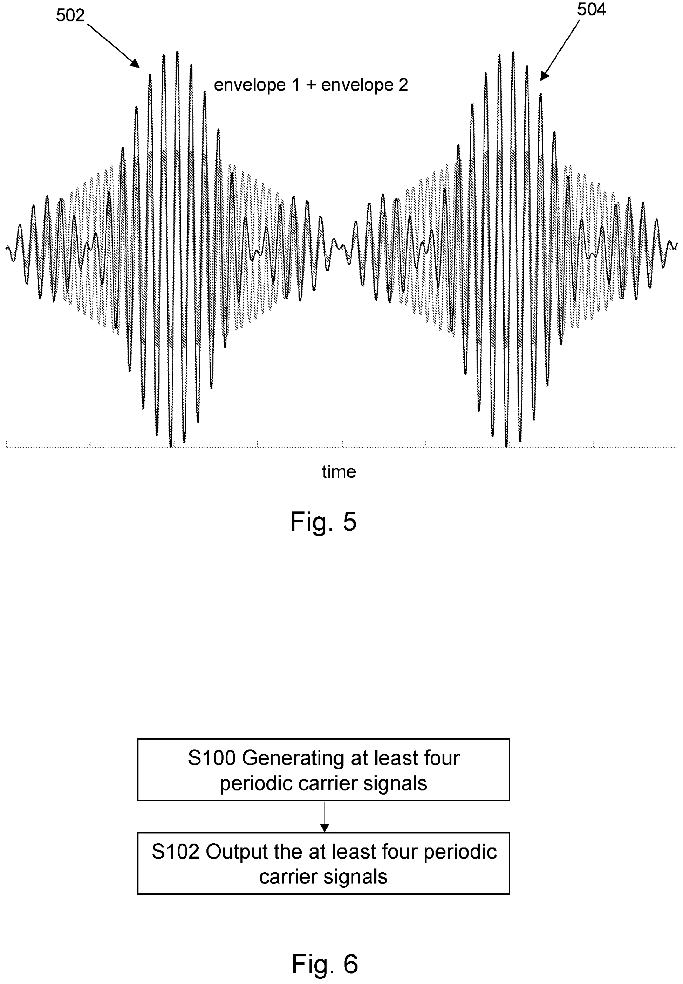

Referring to Fig.5, when two envelope signals of the same frequency are superimposed, the amplitude of the superimposed envelope signal increases significantly, which is shown by part 502 and 504.

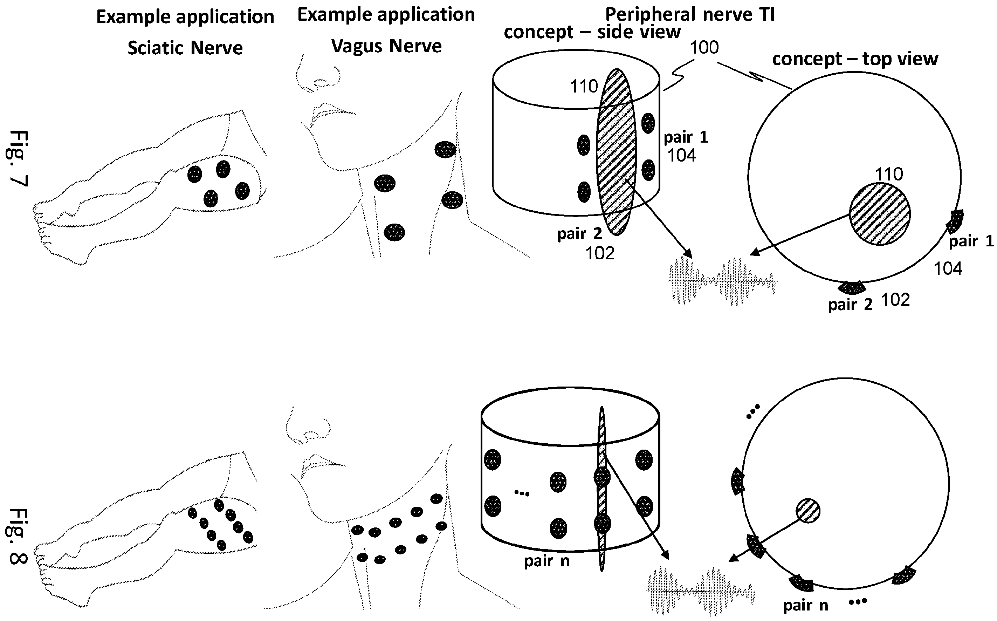

Referring now to Fig. 7, which shows application examples of a target area in form of a sciatic nerve and vagus nerve using two electrode pairs. The periodic carrier signals have temporal interference and generate an envelope signal. The electric field of the envelope signal is the shaded region 110. The frequency of the envelope signal is equal to the frequency difference between the periodic carrier signals. For example, the frequency of a carrier signal of 1000 Hz from one pair of electrodes and the frequency of the carrier signal of 1010 Hz from the other pair of electrodes create the frequency of the envelope signal, which in this case is equal to 10 Hz.

Similarly, referring to Fig. 8, the same application examples are used but with additional pairs of electrodes with periodic carrier signals having temporal interference and generating an envelope signal. For example, in addition to the pervious pairs of electrodes with periodic carrier signals having a frequency of 1000 Hz and 1010 Hz, a periodic carrier signal with a frequency of 2000 Hz from an additional pair of electrodes and a periodic carrier signal with a frequency of 2010 Hz from yet another additional pair of electrodes create the frequency of the envelope signal, which in this case also is equal to 10 Hz. The electric field of the envelope signal is the shaded region 110 in Fig. 8. Where the two envelopes overlap, and a reduced amplitude from the signal generators creates a shaded region which is more focused, as seen in the target cylinder. Additional pairs, up to n pairs, create an increase in focus, i.e. a more narrow shaded region.

The target area can be the patient tissue, such as the sciatic nerve or vagus nerve in the shown examples of Fig. 7 and Fig.8.

When two envelope signals of the same frequency are superimposed, the amplitude of the superimposed envelope signal increases significantly (as seen in Fig. 5), correspondingly the amplitude of stimulation from the generators can be reduced, which allows a narrower target cylinder to be stimulated more efficiently. Comparing to the traditional TI in Fig. 1, the target area is reduced: see target area, the shaded region 110 in Fig. 7 and Fig 8. Consequently, the stimulation within the target cylinder is more accurate, and ideal for PN, when the signal strength output by the electrode pairs is reduced.

In the examples of Fig. 7 and Fig. 8 the frequency difference between any one of the periodic carrier signals 214, 214; 312, 314 is less than 500 Hz, to create an envelope of less than 500 Hz. However, the difference between the mean frequency used to create one envelope and the mean frequency used to create another envelope is ideally greater than or equal to 1kHz, but at least greater than 500 Hz. For example, 1000 Hz and 1010Hz create a 10 Hz envelope and the mean frequency is 1005Hz, 2000 and 2010Hz create another envelope and the mean frequency is 2005Hz, where the difference between the mean frequencies is 1kHz. This is beneficial when using many electrode pairs since if the there are many electrode pairs within a narrow mean frequency difference not only will envelope signals with a desired frequency of 10 Hz be created, but there could also be envelope signals with 20 or 30 Hz which could affect areas outside the target area negatively. With a greater frequency difference envelope signals created between different electrode pairs that are not at the desired frequency will have a large enough frequency not to cause any effect outside the target area.

Since carrier signals with lower strength are utilized in this improved **TI** method, the electric field coverage areas of the periodic carrier signals are smaller, and the coverage area of the superimposed envelope signals become smaller accordingly. Thus, a more accurate stimulation is achieved using the improved TI method.

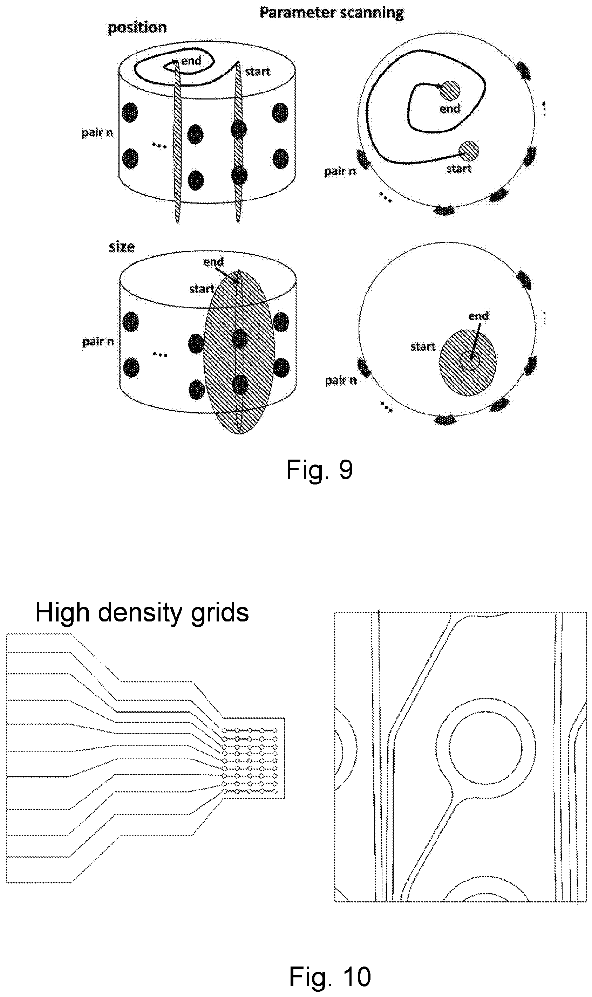

Fig. 9 shows an exemplary embodiment where the electrodes are arranged on the periphery of an imaginary hollow cylinder. Fig. 9 furthermore shows how the accuracy is increasing with increasing number of electrode pairs.

Fig. 10 shows another exemplary embodiment where the pair of electrodes are in form of high-density grids.

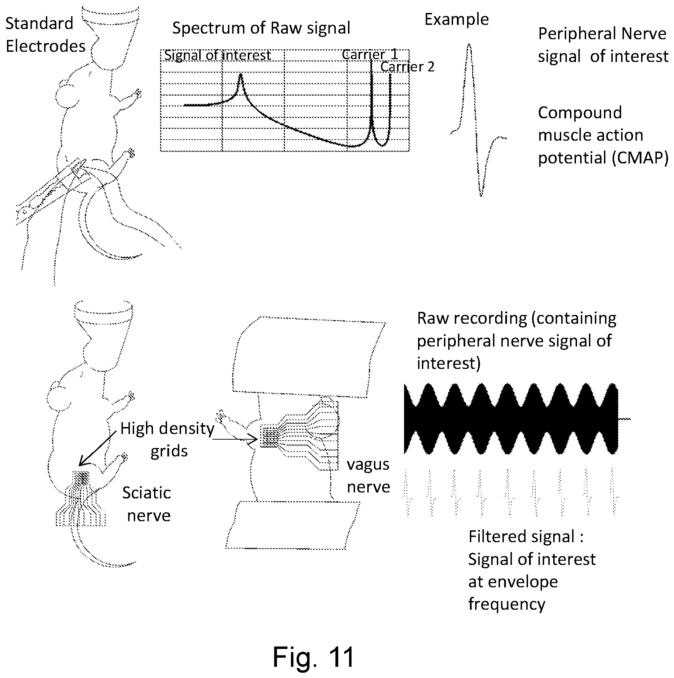

Fig. 11 shows experimental data from tests performed for stimulation of the sciatic nerve and the vagus nerve, both with standard electrodes and high-density grids.

In a preferred embodiment, the tissue being stimulated is a peripheral nerve.

In another preferred embodiment, the peripheral nerve is the vagus nerve.

In yet another preferred embodiment, the frequency of the envelope signals 320 and 322 are within a range of 0.001-500 Hz respectively.

In another preferred embodiment, the frequency of the periodic carrier signals 312, 314, 316 and 318 are within a range of 500 Hz-5 GHz respectively.

In another preferred embodiment, the frequency difference between the periodic carrier signal 312 and 316, and the frequency difference between the periodic carrier signal 314 and 318, is greater than 500 Hz, but preferably greater than 1000 Hz respectively. Because the temporal interference between the periodic carrier signals 312 and 316, 314 and 318 are, as mentioned above, inevitable. To avoid that these unwanted envelope signals disturb normal stimulation, having a frequency difference of the mean frequency used to create one envelope that is greater than 1000Hz leads to that the unwanted envelope signals also have frequencies greater than 1000Hz. These envelope signals with mean frequencies greater than 1000Hz would not perform effective stimulation on a human body, so they would not disturb normal stimulation.

In another preferred embodiment, if there are more than four pairs of electrodes, e.g., 6, 8, 10... pairs of electrodes, envelope signals are generated for each of every two pairs of electrodes. The generated envelope signals have a common envelop frequency equal to envelope signals 320 and 322. The additional generated signals are partially superimposed to the superimposed part 324, and the common superimposed part of all the envelope signals defines the targeting signal used to stimulate the target area and we get an even more accurately defined target area.

In another preferred embodiment, as shown in Fig. 8, the at least four pairs of electrodes are positioned on the periphery of an imaginary hollow cylinder, with each pair of electrodes positioned in one imaginary column on the periphery of the imaginary hollow cylinder.

In another preferred embodiment, as shown in Fig. 8, the imaginary hollow cylinder corresponds to a neck or a thigh of the patient.

In other exemplary embodiments, the nerve stimulated is the phrenic nerve, the hypoglossal nerve, the peroneal nerve, or the sacral nerve.

Turning now to Fig. 6, and referring to Fig. 2 and 3, the method according to the present invention will be described. The method for stimulating a target area by means of a targeting signal 324 is performed by an electronic device as described above. In step S100 the method generates, by means of each of the at least four periodic signal generators 232, 234, 236 and 238 a total of at least four periodic carrier signals 212, 214, 216 and 218; 312, 314, 316 and 318 wherein the frequency difference between any one of the periodic carrier signal frequencies used to create an envelope 212, 214, 216 and 218; 312, 314, 316 and 318 is less than 500 Hz, to create an envelope less than 500 Hz. However, the difference between the mean frequency used to create one envelope and the mean frequency used to create another envelope is ideally greater than or equal to 1kHz, but at least greater than 500 Hz. In step S102 the method outputs, by means of each pair of the at least four pairs of electrodes 202, 204, 206 and 208; 302, 304, 306 and 308 the at least four periodic carrier signals, i.e., at least a first 212; 312, a second 214; 314, a third 216; 316 and a fourth 281; 318 periodic carrier signal; wherein, the first periodic carrier signal 212; 312 output by the first pair of electrodes 202; 302 has a first carrier frequency, the second periodic carrier signal 214; 314 output by the second pair of electrodes 204; 304 has a second carrier frequency, the third periodic carrier signal 216; 316 output by the third pair of electrodes 206; 306 has a third carrier frequency, the fourth periodic carrier signal 218; 318 output by the fourth pair of electrodes 208; 308 has a fourth carrier frequency, the first periodic carrier signal 212; 312 and the second periodic carrier signal 214; 314 causing temporal interference, and generating a first envelope signal 320 with a first envelope frequency, wherein said first envelope frequency is equal to the frequency difference between the first carrier frequency and the second carrier frequency, the third periodic carrier signal 216; 316 and the fourth periodic carrier signal 218; 318 causing temporal interference and generating a second envelope signal 322 with a second envelope frequency, wherein said second envelope frequency is equal to the frequency difference between the third carrier frequency and the fourth carrier frequency, and equal to the first envelope frequency, said at least four pairs of electrodes 202, 204, 206, 208; 302, 304, 306, 308 are positioned in such a way that the first envelope signal 320 and the second envelope signal 322 are at least partially superimposed, and the superimposed part of the envelope signals 320, 322 defines the targeting signal 324 used to stimulate the target area.

In an exemplary embodiment there is provided a method for stimulating a patient tissue by means of a targeting signal, the method is performed by an electronic device, the electronic device comprising at least four pairs of electrodes, i.e. at least a first, a second, a third and a fourth pair of electrodes and at least four signal generators, i.e. at least a first, a second, a third and a fourth signal generator, wherein each pair of electrodes is connected with one of the at least four signal generators, the method comprises:
generating, by means of each of the at least four periodic signal generators a total of at least four periodic carrier signals;
outputting, by means of each pair of the at least four pairs of electrodes, the at least four periodic carrier signals, i.e., at least a first, a second, a third and a fourth periodic carrier signal; wherein,
the first periodic carrier signal output by the first pair of electrodes has a first carrier frequency, the second periodic carrier signal output by the second pair of electrodes has a second carrier frequency, the third periodic carrier signal output by the third pair of electrodes has a third carrier frequency, the fourth periodic carrier signal output by the fourth pair of electrodes has a fourth carrier frequency,
the first periodic carrier signal and the second periodic carrier signal causing temporal interference, and generating a first envelope signal with a first envelope frequency, wherein said first envelope frequency is equal to the frequency difference between the first carrier frequency and the second carrier frequency,
the third periodic carrier signal and the fourth periodic carrier signal causing temporal interference, and generating a second envelope signal with a second envelope frequency, wherein said second envelope frequency is equal to the frequency difference between the third carrier frequency and the fourth carrier frequency, and equal to the first envelope frequency,
the first envelope signal and the second envelope signal are at least partially superimposed, and the superimposed part of the envelope signals defines the targeting signal used to stimulate the patient tissue.

An exemplary embodiment of the method as described above, wherein the first envelope frequency and the second envelope frequency are within a range of 0.001-500 Hz, respectively.

Another exemplary embodiment of the method as described above, wherein the first carrier frequency, the second carrier frequency, the third carrier frequency and the fourth carrier frequency are within a range of 500 Hz-5 GHz respectively.

Yet another exemplary embodiment of the method as described above, wherein the difference between the mean carrier frequency used to create and envelope and the mean carrier frequency used to create another envelope is greater than 500 Hz, preferably greater than 1000Hz.

Another exemplary embodiment of the method as described above, wherein additional electrode pairs other than the first to fourth electrode pair, generate envelope signals for each of every two pairs of electrodes, wherein the generated envelope signals have a common envelope frequency equal to the first envelope frequency and the second envelope frequency, the generated envelope signals are at least partially superimposed to the superimposed part of the first and second envelope signal, the common superimposed part of all the envelope signals defines the targeting signal used to stimulate the target area.

The exemplary method as described above, wherein the tissue is a peripheral nerve.

The exemplary method as described above, wherein the peripheral nerve is the vagus nerve.

The exemplary method as described above, wherein the at least four pairs of electrodes are positioned on the periphery of an imaginary hollow cylinder with each pair of electrodes positioned in one imaginary column on the periphery of the imaginary hollow cylinder.

The exemplary method as described above, wherein the imaginary hollow cylinder corresponds to a neck of the patient.

The exemplary method as described above, wherein the stimulating is used for diagnosis and/or treatment of obesity, depression, epilepsy, inflammatory conditions such as Crohn's disease.

Although the description above contains a plurality of specificities, these should not be construed as limiting the scope of the concept described herein but as merely providing illustrations of some exemplifying embodiments of the described concept. It will be appreciated that the scope of the presently described concept fully encompasses other embodiments which may become obvious to those skilled in the art, and that the scope of the presently described concept is accordingly not to be limited thereto. Reference to an element in the singular is not intended to mean "one and only one" unless explicitly so stated, but rather **"one or more."** Moreover, it is not necessary for an apparatus or method to address each and every problem sought to be solved by the presently described concept, for it to be encompassed hereby. In the exemplary figures, a broken line generally signifies that the feature within the broken line is optional. The invention is defined in the appended claims. Aspects, embodiments and examples disclosed herein which do not fall within the scope of the appended claims do not form part of the invention and are merely provided for illustrative purposes. Furthermore, the methods presented in the present description are not claimed as such but are useful for understanding of the claimed subject matter, in particular since the methods can be performed by the claimed subject-matter.

## Claims

1. An electronic device for stimulating a target area of a peripheral nerve by means of a targeting signal (324), the electronic device comprising at least four pairs of electrodes comprising at least a first (202; 302), a second (204; 304), a third (206; 306) and a fourth (208; 308) pair of electrodes and at least four signal generators comprising at least a first (232), a second (234), a third (236) and a fourth (238) signal generator, wherein each pair of electrodes (202, 204, 206, 208; 302, 304, 306, 308) is connected to one of the at least four signal generators (232, 234, 236, 238) and the electronic device is configured to:
generate, by means of each of the at least four signal generators (232, 234, 236, 238) a total of at least four periodic carrier signals (212, 214, 216, 218; 312, 314, 316, 318),
output, by means of each pair of the at least four pairs of electrodes (202, 204, 206, 208; 302, 304, 306, 308), the at least four periodic carrier signals, i.e. at least a first (212; 312), a second (214; 314), a third (216; 316) and a fourth (218; 318) periodic carrier signal; wherein,
the first periodic carrier signal (212; 312) output by the first pair of electrodes (202; 302) has a first carrier frequency, the second periodic carrier signal (214; 314) output by the second pair of electrodes (204; 304) has a second carrier frequency, the third periodic carrier signal (216; 316) output by the third pair of electrodes (206; 306) has a third carrier frequency, the fourth periodic carrier signal (218; 318) output by the fourth pair of electrodes (208; 308) has a fourth carrier frequency,
the first periodic carrier signal (212; 312) and the second periodic carrier signal (214; 314) cause temporal interference, and generate a first envelope signal (320) with a first envelope frequency, wherein said first envelope frequency is equal to the frequency difference between the first carrier frequency and the second carrier frequency,
the third periodic carrier signal (216; 316) and the fourth periodic carrier signal (218; 318) cause temporal interference, and generate a second envelope signal (322) with a second envelope frequency, wherein said second envelope frequency is equal to the frequency difference between the third carrier frequency and the fourth carrier frequency, and equal to the first envelope frequency,
said at least four pairs of electrodes (202, 204, 206, 208; 302, 304, 306, 308) are positioned in such a way that the first envelope signal (320) and the second envelope signal (322) are at least partially superimposed, and the superimposed part of the envelope signals (320, 322) defines the targeting signal (324) used to stimulate the target area of the peripheral nerve.

2. The electronic device as claimed in claim 1, wherein the frequency difference between any one of the periodic carrier signal frequencies is used to create an envelope, the difference between a mean frequency used to create one envelope and a mean frequency used to create another envelope is greater than 500 Hz, preferably greater than 1kHz.

3. The electronic device as claimed in claim 1 or 2, wherein the first envelope frequency and the second envelope frequency are within a range of 0.001-500 Hz, respectively.

4. The electronic device as claimed in any one of claims 1 to 3, wherein the first carrier frequency, the second carrier frequency, the third carrier frequency and the fourth carrier frequency are within a range of 500 Hz-5 GHz respectively.

5. The electronic device as claimed in any one of claims 1 to 4, wherein one or more additional electrode pairs other than the first to fourth electrode pair, are configured to generate respective envelope signals for each of the additional pairs of electrodes, wherein the generated envelope signals have a common envelope frequency equal to the first envelope frequency and the second envelope frequency, wherein
the generated envelope signals are at least partially superimposed to the superimposed part of the first and second envelope signal (320, 322), and wherein
the common superimposed part of all the envelope signals defines the targeting signal (324) used to stimulate the target area of the peripheral nerve.

6. The electronic device according to any one of claims 1 to 5, wherein the target area of the peripheral nerve has the shape of a target cylinder.

## Patentansprüche

1. Elektronische Vorrichtung zur Stimulation eines Zielbereichs eines peripheren Nervs mittels eines Zielsignals (324), wobei die elektronische Vorrichtung mindestens vier Elektrodenpaare umfasst, die mindestens ein erstes (202; 302), ein zweites (204; 304), ein drittes (206; 306) und ein viertes (208; 308) Elektrodenpaar umfassen, sowie mindestens vier Signalgeneratoren, die mindestens einen ersten (232), einen zweiten (234), einen dritten (236) und einen vierten (238) Signalgenerator umfassen, wobei jedes Elektrodenpaar (202, 204, 206, 208; 302, 304, 306, 308) mit einem der mindestens vier Signalgeneratoren (232, 234, 236, 238) verbunden ist, und die elektronische Vorrichtung konfiguriert ist zum:
Generieren, mittels jedes der mindestens vier Signalgeneratoren (232, 234, 236, 238), von insgesamt mindestens vier periodischen Trägersignalen (212, 214, 216, 218; 312, 314, 316, 318),
Ausgeben, mittels jedes der mindestens vier Elektrodenpaare (202, 204, 206, 208; 302, 304, 306, 308), der mindestens vier periodischen Trägersignale, d. h. mindestens eines ersten (212; 312), eines zweiten (214; 314), eines dritten (216; 316) und eines vierten (218; 318) periodischen Trägersignals; wobei
das erste periodische Trägersignal (212; 312), das vom ersten Elektrodenpaar (202; 302) ausgegeben wird, eine erste Trägerfrequenz aufweist, das zweite periodische Trägersignal (214; 314), das vom zweiten Elektrodenpaar (204; 304) ausgegeben wird, eine zweite Trägerfrequenz aufweist, das dritte periodische Trägersignal (216; 316), das vom dritten Elektrodenpaar (206; 306) ausgegeben wird, eine dritte Trägerfrequenz aufweist, das vierte periodische Trägersignal (218; 318), das vom vierten Elektrodenpaar (208; 308) ausgegeben wird, eine vierte Trägerfrequenz aufweist,
wobei das erste periodische Trägersignal (212; 312) und das zweite periodische Trägersignal (214; 314) zeitliche Interferenzen bewirken und ein erstes Hüllkurvensignal (320) mit einer ersten Hüllkurvenfrequenz generieren, wobei die erste Hüllkurvenfrequenz gleich der Frequenzdifferenz zwischen der ersten Trägerfrequenz und der zweiten Trägerfrequenz ist,
wobei das dritte periodische Trägersignal (216; 316) und das vierte periodische Trägersignal (218; 318) zeitliche Interferenzen bewirken und ein zweites Hüllkurvensignal (322) mit einer zweiten Hüllkurvenfrequenz generieren, wobei die zweite Hüllkurvenfrequenz gleich der Frequenzdifferenz zwischen der dritten Trägerfrequenz und der vierten Trägerfrequenz und gleich der ersten Hüllkurvenfrequenz ist,
wobei die mindestens vier Elektrodenpaare (202, 204, 206, 208; 302, 304, 306, 308) so positioniert, dass sich das erste Hüllkurvensignal (320) und das zweite Hüllkurvensignal (322) mindestens teilweise überlagern und der überlagerte Teil der Hüllkurvensignale (320, 322) das Zielsignal (324) definiert, das zur Stimulation des Zielbereichs des peripheren Nervs verwendet wird.

2. Elektronische Vorrichtung nach Anspruch 1, wobei die Frequenzdifferenz zwischen einer beliebigen periodischen Trägersignalfrequenz zur Erzeugung einer Hüllkurve verwendet wird, die Differenz zwischen einer mittleren Frequenz, die zur Erzeugung einer Hüllkurve verwendet wird, und einer mittleren Frequenz, die zur Erzeugung einer anderen Hüllkurve verwendet wird, größer als 500 Hz, bevorzugt größer als 1 kHz ist.

3. Elektronische Vorrichtung nach Anspruch 1 oder 2, wobei die erste Hüllkurvenfrequenz und die zweite Hüllkurvenfrequenz jeweils in einem Bereich von 0,001 - 500 Hz liegen.

4. Elektronische Vorrichtung nach einem der Ansprüche 1 bis 3, wobei die erste Trägerfrequenz, die zweite Trägerfrequenz, die dritte Trägerfrequenz und die vierte Trägerfrequenz jeweils im Bereich von 500 Hz - 5 GHz liegen.

5. Elektronische Vorrichtung nach einem der Ansprüche 1 bis 4, wobei ein oder mehrere zusätzliche Elektrodenpaare, die nicht das erste bis vierte Elektrodenpaar sind, konfiguriert sind, für jedes der zusätzlichen Elektrodenpaare jeweilige Hüllkurvensignale zu generieren, wobei die generierten Hüllkurvensignale eine gemeinsame Hüllkurvenfrequenz aufweisen, die gleich der ersten Hüllkurvenfrequenz und der zweiten Hüllkurvenfrequenz ist, wobei die generierten Hüllkurvensignale mindestens teilweise mit dem überlagerten Teil des ersten und zweiten Hüllkurvensignals (320, 322) überlagert sind und wobei der gemeinsame überlagerte Teil aller Hüllkurvensignale das Zielsignal (324) definiert, das zur Stimulation des Zielbereichs des peripheren Nervs verwendet wird.

6. Elektronische Vorrichtung nach einem der Ansprüche 1 bis 5, wobei der Zielbereich des peripheren Nervs die Form eines Zielzylinders aufweist.

## Revendications

1. Dispositif électronique de stimulation d'une zone cible d'un nerf périphérique au moyen d'un signal de ciblage (324), le dispositif électronique comprenant au moins quatre paires d'électrodes comprenant au moins une première (202 ; 302), une deuxième (204 ; 304), une troisième (206 ; 306) et une quatrième (208 ; 308) paire d'électrodes et au moins quatre générateurs de signaux comprenant au moins un premier (232), un deuxième (234), un troisième (236) et un quatrième (238) générateur de signaux, dans lequel chaque paire d'électrodes (202, 204, 206, 208 ; 302, 304, 306, 308) est raccordée à l'un des au moins quatre générateurs de signaux (232, 234, 236, 238) et le dispositif électronique est configuré pour :
générer, au moyen de chacun des au moins quatre générateurs de signaux (232, 234, 236, 238), un total d'au moins quatre signaux porteurs périodiques (212, 214, 216, 218 ; 312, 314, 316, 318),
émettre, au moyen de chaque paire des au moins quatre paires d'électrodes (202, 204, 206, 208 ; 302, 304, 306, 308), au moins quatre signaux porteurs périodiques, à savoir au moins un premier (212 ; 312), un deuxième (214 ; 314), un troisième (216 ; 316) et un quatrième (218 ; 318) signal porteur périodique ; dans lequel,
le premier signal porteur périodique (212 ; 312) émis par la première paire d'électrodes (202 ; 302) présente une première fréquence porteuse, le deuxième signal porteur périodique (214 ; 314) émis par la deuxième paire d'électrodes (204 ; 304) présente une deuxième fréquence porteuse, le troisième signal porteur périodique (216 ; 316) émis par la troisième paire d'électrodes (206; 306) présente une troisième fréquence porteuse, le quatrième signal porteur périodique (218 ; 318) émis par la quatrième paire d'électrodes (208 ; 308) présente une quatrième fréquence porteuse,
le premier signal porteur périodique (212 ; 312) et le deuxième signal porteur périodique (214 ; 314) provoquent une interférence temporelle et génèrent un premier signal d'enveloppe (320) avec une première fréquence d'enveloppe, dans lequel la première fréquence d'enveloppe est égale à la différence de fréquence entre la première fréquence porteuse et la deuxième fréquence porteuse.
le troisième signal porteur périodique (216 ; 316) et le quatrième signal porteur périodique (218 ; 318) provoquent une interférence temporelle et génèrent un second signal d'enveloppe (322) avec une seconde fréquence d'enveloppe, dans lequel la seconde fréquence d'enveloppe est égale à la différence de fréquence entre la troisième fréquence porteuse et la quatrième fréquence porteuse, et égale à la première fréquence d'enveloppe.
lesdites au moins quatre paires d'électrodes (202, 204, 206, 208 ; 302, 304, 306, 308) sont positionnées de telle manière que le premier signal d'enveloppe (320) et le second signal d'enveloppe (322) soient au moins partiellement superposés, et que la partie superposée des signaux d'enveloppe (320, 322) définisse le signal de ciblage (324) utilisé pour stimuler la zone cible du nerf périphérique.

2. Dispositif électronique selon la revendication 1, dans lequel la différence de fréquence entre l'une quelconque des fréquences de signal porteur périodique est utilisée pour créer une enveloppe, la différence entre une fréquence moyenne utilisée pour créer une enveloppe et une fréquence moyenne utilisée pour créer une autre enveloppe est supérieure à 500 Hz, de préférence supérieure à 1 kHz.

3. Dispositif électronique selon la revendication 1 ou 2, dans lequel la première fréquence d'enveloppe et la seconde fréquence d'enveloppe sont dans une plage de 0,001-500 Hz, respectivement.

4. Dispositif électronique selon l'une quelconque des revendications 1 à 3, dans lequel la première fréquence porteuse, la deuxième fréquence porteuse, la troisième fréquence porteuse et la quatrième fréquence porteuse sont dans une plage de 500 Hz-5 GHz, respectivement.

5. Dispositif électronique selon l'une quelconque des revendications 1 à 4, dans lequel une ou plusieurs paires d'électrodes supplémentaires autres que la première à la quatrième paire d'électrodes, sont configurées pour générer des signaux d'enveloppe respectifs pour chacune des paires supplémentaires d'électrodes, dans lequel les signaux d'enveloppe générés présentent une fréquence d'enveloppe commune égale à la première fréquence d'enveloppe et à la seconde fréquence d'enveloppe, dans lequel les signaux d'enveloppe générés sont au moins partiellement superposés à la partie superposée du premier et du second signal d'enveloppe (320, 322) et dans lequel la partie superposée commune de tous les signaux d'enveloppe définit le signal de ciblage (324) utilisé pour stimuler la zone cible du nerf périphérique.

6. Dispositif électronique selon l'une quelconque des revendications 1 à 5, dans lequel la zone cible du nerf périphérique présente la forme d'un cylindre cible.
